# EUROPEAN PATENT SPECIFICATION

(11) **EP 1 509 606 B1**
(45) Date of publication and mention of the grant of the patent: **31.10.2007**
(21) Application number: 03735524.5
(22) Date of filing: 03.06.2003
(51) Int. Cl.: C12N 15/29, C07K 14/415, C07K 16/16, A61K 39/36

(54) **ALLERGEN FROM MUGWORT POLLEN**
ALLERGEN AUS BEIFUSS-POLLEN
ALLERGENE DU POLLEN D'ARMOISE

(30) Priority: 04.06.2002 EP 02012302
(43) Date of publication of application: 02.03.2005
(73) Proprietor: BIOMAY Produktions- und Handels- Aktiengesellschaft, 1030 Wien (AT)
(72) Inventor: FERREIRA, Fátima, A-5020 Salzburg (AT); HUBINGER, Gudrun, A-4852 Weyregg am Attersee (AT); EBNER, Christof, A-2345 Brunn am Gebirge (AT); RICHTER, Klaus, A-5081 Anif (AT)
(74) Representative: Keller, Günter
(86) International application number: PCT/EP2003/005780
(87) International publication number: WO 2003/102189

(56) References cited:
- WO-A-94/01560
- WO-A-96/13589
- WO-A-99/49045
- FERREIRA FATIMA ET AL: "Isolation and characterization of cDNA clones coding for mugwort (Artemisia vulgaris) pollen allergens." INTERNATIONAL ARCHIVES OF ALLERGY AND IMMUNOLOGY, vol. 124, no. 1-3, January 2001 (2001-01), pages 77-79, XP008006228 ISSN: 1018-2438
- HIRSCHWEHR R ET AL: "ALLERGENS, IGE, MEDIATORS, INFLAMMATORY MECHANISMS. IDENTIFICATION OF COMMON ALLERGENIC STRUCTURES IN MUGWORT AND RAGWEED POLLEN" JOURNAL OF ALLERGY AND CLINICAL IMMUNOLOGY, MOSBY - YEARLY BOOK, INC, US, vol. 101, no. 2, PART 1, February 1998 (1998-02), pages 196-206, XP000857295 ISSN: 0091-6749 cited in the application
- HILZ H. ET AL: 'STIMULATION OF PROTEINASE K ACTION BY DENATURING AGENTS APPLICATION TO THE ISOLATION OF NUCLEIC-ACIDS AND THE DEGRADATION OF MASKED PROTEINS' EUROPEAN JOURNAL OF BIOCHEMISTRY vol. 56, 1975, pages 103 - 108, XP008060213
- PERTL H. ET AL: 'Molecular and physiological characterisation of a 14-3-3 protein from lily pollen grains regulating the activity of the plasma membrane H+ ATPase during pollen grain germination and tube growth' PLANTA vol. 213, no. 1, 2001, pages 132 - 141
- WOPFNER N. ET AL: 'Molecular and immunological characterization of profilin from mugwort pollen' BIOL CHEM vol. 383, 2002, pages 1779 - 1789, XP008060183

### SEQUENCE LISTING

<110> Biomay Produktions- und Handels-Aktiengesellschaft
   <120> Allergenic protein from mugwort pollen
   <130> Allergenic protein from mugwort pollen
<140>
   <141>
<160> 10
<170> PatentIn ver. 2.1
<210> 1
   <211> 396
   <212> PRT
   <213> Artemisia vulgaris
<400> 1
   Met Glu Lys His Tyr Phe Val Ile Leu Phe Thr Ala Ala Phe Val Phe
   1 5 10 15
   Val Gly Ala Ala Ala Arg Ala Asp Ile Gly Asp Glu Leu Glu Ala Ala
   20 25 30
   Gln Phe Asn Ser Thr Arg Arg Gly Leu His Glu Cys Ala Ala His Asn
   35 40 45
   Ile Ile Asp Lys Cys Trp Arg Cys Lys Ala Asp Trp Glu Lys Asn Arg
   50 55 60
   Gln Ala Leu Ala Lys Cys Ala Gln Gly Phe Ala Lys Gly Thr Thr Gly
   65 70 75 80
   Gly Leu Gly Gly Glu Ile Tyr Val Val Thr Asp Cys Ser Asp Asp Asn
   85 90 95
   Ala Ala Asn Pro Lys Pro Gly Thr Leu Arg Cys Gly Val Thr Gln Asp
   100 105 110
   Lys Pro Leu Trp Ile Ile Phe Lys Lys Asp Met Val Ile Lys Leu Lys
   115 120 125
   His Glu Leu Val Ile Asn Lys Asp Lys Thr Ile Asp Gly Arg Gly Ala
   130 135 140
   Asn Val Glu Ile Thr Cys Gly Gly Leu Thr Ile His Asn Val Cys Asn
   145 150 155 160
   Val Ile Ile His Asn Ile His Ile His Asp Ile Lys Val Thr Glu Gly
   165 170 175
   Gly Ile Ile Lys Ala Thr Asp Ala Lys Pro Gly His Arg His Lys Ser
   180 185 190
   Asp Gly Asp Gly Ile Cys Val Ala Gly Ser Ser Lys Ile Trp Ile Asp
   195 200 205
   His Cys Thr Leu Ser His Gly Pro Asp Gly Leu Ile Asp Val Thr Leu
   210 215 220
   Gly Ser Thr Ala Val Thr Ile Ser Asn Cys Lys Phe Ser His His Gln
   225 230 235 240
   Lys Ile Leu Leu Leu Gly Ala Asp Asn Ser His Val Asp Asp Lys Lys
   245 250 255
   Met His Val Thr Val Ala Phe Asn Arg Phe Ala Glu Ala Cys Asp Gln
               260                 265                 270
   Arg Met Pro Arg Cys Arg Phe Gly Phe Phe Gln Val Val Asn Asn Asp
           275                 280                 285
   Tyr Thr Ser Trp Gly Thr Tyr Ala Ile Gly Gly Ser Ala Asn Pro Thr
       290                 295                 300
   Ile Leu Ser Gln Gly Asn Arg Phe His Ala Pro Asn Asp Pro Met Lys
   305                 310                 315                 320
   Lys Asn Val Leu Val Arg Ala Asp Ala Pro His Thr Glu Ser Met Lys
                   325                 330                 335
   Trp Asn Trp Arg Ser Glu Lys Asp Leu Leu Glu Asn Gly Ala Ile Phe
               340                 345                 350
   Val Ala Ser Gly Cys Asp Pro His Leu Thr Pro Glu Gln Lys Ser His
           355                 360                 365
   Leu Ile Pro Ala Glu Pro Gly Ser Ala Val Leu Gln Leu Thr Ser Cys
       370                 375                 380
   Ala Gly Thr Leu Lys Cys Val Pro Gly Lys Pro Cys
   385                 390                 395
<210> 2
   <211> 1188
   <212> DNA
   <213> Artemisia vulgaris
<400> 2
   atggaaaaac attattttgt tatattgttc accgcagcgt ttgttttcgt gggtgcagct 60
   gctcgggctg acattggtga tgagctcgaa gcggctcaat ttaattcaac aaggaggggc 120
   ttacacgaat gtgcagcaca taacataata gacaagtgtt ggaggtgcaa agctgattgg 180
   gaaaaaaacc gacaagcatt agccaaatgc gcgcaaggtt ttgcaaaggg aacaactggc 240
   ggattgggag gggaaatcta cgtggtgact gattgttcag atgacaatgc tgcaaatcca 300
   aagccaggga cacttcgttg tggtgtcacc caagataaac ctttgtggat catctttaag 360
   aaagatatgg tcataaaact taaacacgag cttgtgataa acaaagacaa gacaattgat 420
   ggaagaggtg caaatgttga gatcacttgt ggcggtctca ccattcacaa cgtttgcaat 480
   gtgatcattc ataacattca catacatgat attaaagtaa ccgaaggtgg aattattaag 540
   gcaacggacg ctaaaccagg acatagacat aagagcgacg gagatggtat ttgtgttgct 600
   ggttcttcaa agatatggat cgatcattgc acacttagtc atggtccaga tggccttatt 660
   gatgtcacgt tgggtagcac agccgttacc atttccaatt gcaaatttag ccatcaccaa 720
   aaaattctat tactcggagc agacaattca catgtagacg ataaaaaaat gcatgtcaca 780
   gtagcattca acaggttcgc agaagcatgt gatcaaagaa tgccacgatg tcgatttgga 840
   tttttccaag ttgttaacaa tgactacacc agctggggaa cgtacgccat tggtggtagt 900
   gccaatccta ctatccttag ccaaggcaac cgattccatg ctccgaatga cccaatgaag 960
   aaaaatgtgt tggtgagggc tgatgcacca catacagagt caatgaagtg gaattggaga 1020
   tctgagaaag acttgttaga aaatggagct atatttgtag catcagggtg cgacccgcat 1080
   ctaaccccgg aacaaaaaag ccatttgatt ccagctgaac caggatcagc agttcttcaa 1140
   ctcaccagtt gtgctggcac gctcaaatgc gttcctggaa aaccttgt             1188
<210> 3
   <211> 1188
   <212> DNA
   <213> Artemisia vulgaris
<400> 3
   atggaaaaac attattttgt tatattgttc accgcagcgt ttgttttcgt gggtgcagct 60
   gctcgggctg acattggtga tgagctcgaa gcggctcaat ttaattcaac aaggaggggc 120
   ttacacgaat gtgcagcaca taacataata gacaagtgtt ggaggtgcaa agctgattgg 180
   gaaaaaaacc gacaagcatt agccaaatgc gcgcaaggtt ttgcaaaggg aacaactggc 240
   ggattgggag gggaaatcta cgtggtgact gattgttcag atgacaatgc tgcaaatcca 300
   aagccaggga cacttcgttg tggtgtcacc caagataaac ctttgtggat catcttcaag 360
   aaagatatgg tcataaaact taaacacgag cttgtgataa acaaagacaa gacaattgat 420
   ggaagaggtg caaatgttga gatcacttgt ggcggtctca ccattcacaa cgtttgcaat 480
   gtgatcattc ataacattca catacatgat attaaagtaa cggaaggtgg aattattaag 540
   gcaacggacg ctaaaccagg gcatagacat aagagcgacg gagatggtat ttgtgttgct 600
   ggttcttcga agatatggat cgatcattgc acacttagtc atggtccaga tggccttatt 660
   gatgtcacgt tgggtagcac agccgttacc atttccaatt gcaaatttag ccatcaccaa 720
   aaaattctat tactcggagc agacaattca catgtagacg ataaaaaaat gcatgtcaca 780
   gtcgcattca acaggttcgc agaagcatgt gatcaaagaa tgccacgatg tcgatttgga 840
   tttttccaag ttgttaacaa tgactacacc agctggggaa cgtacgccat tggtggtagc 900
   gccaatccta ctatccttag ccaaggcaac cgattccatg ctcccaatga cccaatgaag 960
   aaaaatgtgt tggtgagggc tgatgcacca catacagagt caatgaagtg gaattggaga 1020
   tctgagaaag acttgttaga aaatggagct atatttgtag catcagggtg cgacccgcat 1080
   ctaaccccgg aacaaaaaag ccatttaatt ccagctgaac caggatcagc agttcttcaa 1140
   ctcaccagtt gtgctggcac gctcaaatgc gttcctggaa aaccttgt             1188
<210> 4
   <211> 41
   <212> DNA
   <213> Artificial Sequence
<220>
   <223> Description of Artificial sequence: Primer
<400> 4
   gagagagacc atggctcggg ctgacattgg tgatgagctc g 41
<210> 5
   <211> 42
   <212> DNA
   <213> Artificial Sequence
<220>
   <223> Description of Artificial sequence: Primer
<400> 5
   gagagagact cgagttaaca aggttttcca ggaacgcatt tg 42
<210> 6
   <211> 1523
   <212> DNA
   <213> Artemisia vulgaris
<400> 6
   ggaattcggc acgagaaatc tacaaaaatt gataaaaata aaaataacaa agcgagtcat 60
   tggcttgcat acatggctac atgattcgct ttagacaaca caataaataa tcagcaatat 120
   ataaagtacc ttcggtactt tgagatagaa agttgtaaaa aaagataatc atacaataca 180
   atggaaaaac attattttgt tatattgttc accgcagcgt ttgttttcgt gggtgcagct 240
   gctcgggctg acattggtga tgagctcgaa gcggctcaat ttaattcaac aaggaggggc 300
   ttacacgaat gtgcagcaca taacataata gacaagtgtt ggaggtgcaa agctgattgg 360
   gaaaaaaacc gacaagcatt agccaaatgc gcgcaaggtt ttgcaaaggg aacaactggc 420
   ggattgggag gggaaatcta cgtggtgact gattgttcag atgacaatgc tgcaaatcca 480
   aagccaggga cacttcgttg tggtgtcacc caagataaac ctttgtggat catctttaag 540
   aaagatatgg tcataaaact taaacacgag cttgtgataa acaaagacaa gacaattgat 600
   ggaagaggtg caaatgttga gatcacttgt ggcggtctca ccattcacaa cgtttgcaat 660
   gtgatcattc ataacattca catacatgat attaaagtaa ccgaaggtgg aattattaag 720
   gcaacggacg ctaaaccagg acatagacat aagagcgacg gagatggtat ttgtgttgct 780
   ggttcttcaa agatatggat cgatcattgc acacttagtc atggtccaga tggccttatt 840
   gatgtcacgt tgggtagcac agccgttacc atttccaatt gcaaatttag ccatcaccaa 900
   aaaattctat tactcggagc agacaattca catgtagacg ataaaaaaat gcatgtcaca 960
   gtagcattca acaggttcgc agaagcatgt gatcaaagaa tgccacgatg tcgatttgga 1020
   tttttccaag ttgttaacaa tgactacacc agctggggaa cgtacgccat tggtggtagt 1080
   gccaatccta ctatccttag ccaaggcaac cgattccatg ctccgaatga cccaatgaag 1140
   aaaaatgtgt tggtgagggc tgatgcacca catacagagt caatgaagtg gaattggaga 1200
   tctgagaaag acttgttaga aaatggagct atatttgtag catcagggtg cgacccgcat 1260
   ctaaccccgg aacaaaaaag ccatttgatt ccagctgaac caggatcagc agttcttcaa 1320
   ctcaccagtt gtgctggcac gctcaaatgc gttcctggaa aaccttgtta atagttatca 1380
   cccacttttt ttttttattg ttattagcat tttttatact tgttaggatt gtagtggaat 1440
   gagacattga tacgtgcatt acaagaccat tcatcaacat attttgctac tatcacatgt 1500
   tcacgttaaa aaaaaaaaaa aaa                                          1523
<210> 7
   <211> 1513
   <212> DNA
   <213> Artemisia vulgaris
<400> 7
   ggaattcggc acgagaatct acaaaaattg ataaaaataa aaataaaaat aacaaagcga 60
   gtcattggct tgcatacatg gctacatgat tcgctttaga caacacaata aataatcagc 120
   aatatataaa gtaccttcgg tactttgaga tagaaagttg taaaaaaaga taatcataca 180
   atacaatgga aaaacattat tttgttatat tgttcaccgc agcgtttgtt ttcgtgggtg 240
   cagctgctcg ggctgacatt ggtgatgagc tcgaagcggc tcaatttaat tcaacaagga 300
   ggggcttaca cgaatgtgca gcacataaca taatagacaa gtgttggagg tgcaaagctg 360
   attgggaaaa aaaccgacaa gcattagcca aatgcgcgca aggttttgca aagggaacaa 420
   ctggcggatt gggaggggaa atctacgtgg tgactgattg ttcagatgac aatgctgcaa 480
   atccaaagcc agggacactt cgttgtggtg tcacccaaga taaacctttg tggatcatct 540
   ttaagaaaga tatggtcata aaacttaaac acgagcttgt gataaacaaa gacaagacaa 600
   ttgatggaag aggtgcaaat gttgagatca cttgtggcgg tctcaccatt cacaacgttt 660
   gcaatgtgat cattcataac attcacatac atgatattaa agtaaccgaa ggtggaatta 720
   ttaaggcaac ggacgctaaa ccaggacata gacataagag cgacggagat ggtatttgtg 780
   ttgctggttc ttcaaagata tggatcgatc attgcacact tagtcatggt ccagatggcc 840
   ttattgatgt cacgttgggt agcacagccg ttaccatttc caattgcaaa tttagccatc 900
   accaaaaaat tctattactc ggagcagaca attcacatgt agacgataaa aaaatgcatg 960
   tcacagtagc attcaacagg ttcgcagaag catgtgatca aagaatgcca cgatgtcgat 1020
   ttggattttt ccaagttgtt aacaatgact acaccagctg gggaacgtac gccattggtg 1080
   gtagtgccaa tcctactatc cttagccaag gcaaccgatt ccatgctccg aatgacccaa 1140
   tgaagaaaaa tgtgttggtg agggctgatg caccacatac agagtcaatg aagtggaatt 1200
   ggagatctga gaaagacttg ttagaaaatg gagctatatt tgtagcatca gggtgcgacc 1260
   cgcatctaac cccggaacaa aaaagccatt tgattccagc tgaaccagga tcagcagttc 1320
   ttcaactcac cagttgtgct ggcacgctca aatgcgttcc tggaaaacct tgttaatagt 1380
   tatcacccac tttttatttt tattgttatt agcatttttt atacttgtta ggattgtagt 1440
   ggaatgagac attgatacgt gcattacaag accattcatc aacatatttt gctaaaaaaa 1500
   aaaaaaaaaa aaa                                                     1513
<210> 8
   <211> 1520
   <212> DNA
   <213> Artemisia vulgaris
<400> 8
   gcggccgctc tagaactagt ggatcccccg ggctgcagga attcggcacg agaaattgat 60
   aaaaataaaa ataaaaataa caaagcgagt cattggcttg catacatggc tacatgattc 120
   gctttagaca acacaataaa taatcagcaa tatataaagt accttcggta ctttgagata 180
   gaaagttgta aaaaaagata atcatacaat acaatggaaa aacattattt tgttatattg 240
   ttcaccgcag cgtttgtttt cgtgggtgca gctgctcggg ctgacattgg tgatgagctc 300
   gaagcggctc aatttaattc aacaaggagg ggcttacacg aatgtgcagc acataacata 360
   atagacaagt gttggaggtg caaagctgat tgggaaaaaa accgacaagc attagccaaa 420
   tgcgcgcaag gttttgcaaa gggaacaact ggcggattgg gaggggaaat ctacgtggtg 480
   actgattgtt cagatgacaa tgctgcaaat ccaaagccag ggacacttcg ttgtggtgtc 540
   acccaagata aacctttgtg gatcatcttc aagaaagata tggtcataaa acttaaacac 600
   gagcttgtga taaacaaaga caagacaatt gatggaagag gtgcaaatgt tgagatcact 660
   tgtggcggtc tcaccattca caacgtttgc aatgtgatca ttcataacat tcacatacat 720
   gatattaaag taacggaagg tggaattatt aaggcaacgg acgctaaacc agggcataga 780
   cataagagcg acggagatgg tatttgtgtt gctggttctt cgaagatatg gatcgatcat 840
   tgcacactta gtcatggtcc agatggcctt attgatgtca cgttgggtag cacagccgtt 900
   accatttcca attgcaaatt tagccatcac caaaaaattc tattactcgg agcagacaat 960
   tcacatgtag acgataaaaa aatgcatgtc acagtcgcat tcaacaggtt cgcagaagca 1020
   tgtgatcaaa gaatgccacg atgtcgattt ggatttttcc aagttgttaa caatgactac 1080
   accagctggg gaacgtacgc cattggtggt agcgccaatc ctactatcct tagccaaggc 1140
   aaccgattcc atgctcccaa tgacccaatg aagaaaaatg tgttggtgag ggctgatgca 1200
   ccacatacag agtcaatgaa gtggaattgg agatctgaga aagacttgtt agaaaatgga 1260
   gctatatttg tagcatcagg gtgcgacccg catctaaccc cggaacaaaa aagccattta 1320
   attccagctg aaccaggatc agcagttctt caactcacca gttgtgctgg cacgctcaaa 1380
   tgcgttcctg gaaaaccttg ttaatagtta tcacccactt tttattttta ttgttattag 1440
   cattttttat acttgttagg attgtagtgg aatgagacat tgatacgtgc attacaagac 1500
   caaaaaaaaa aaaaaaaaaa 1520
<210> 9
   <211> 1535
   <212> DNA
   <213> Artemisia vulgaris
<400> 9
   ggcggccgct ctagaactag tggatccccc gggctgcagg aattcggcac gagtgaaaat 60
   ctacaaaaat tgataaaaat aaaaataaaa ataacaaagc gagtcattgg cttgcataca 120
   tggctacatg attcgcttta gacaacacaa taaataatca gcaatatata aagtaccttc 180
   ggtactttga gatagaaagt tgtaaaaaaa gataatcata caatacaatg gaaaaacatt 240
   attttgttat attgttcacc gcagcgtttg ttttcgtggg tgcagctgct cgggctgaca 300
   ttggtgatga gctcgaagcg gctcaattta attcaacaag gaggggctta cacgaatgtg 360
   cagcacataa cataatagac aagtgttgga ggtgcaaagc tgattgggaa aaaaaccgac 420
   aagcattagc caaatgcgcg caaggttttg caaagggaac aactggcgga ttgggagggg 480
   aaatctacgt ggtgactgat tgttcagatg acaatgctgc aaatccaaag ccagggacac 540
   ttcgttgtgg tgtcacccaa gataaacctt tgtggatcat ctttaagaaa gatatggtca 600
   taaaacttaa acacgagctt gtgataaaca aagacaagac aattgatgga agaggtgcaa 660
   atgttgagat cacttgtggc ggtctcacca ttcacaacgt ttgcaatgtg atcattcata 720
   acattcacat acatgatatt aaagtaaccg aaggtggaat tattaaggca acggacgcta 780
   aaccaggaca tagacataag agcgacggag atggtatttg tgttgctggt tcttcaaaga 840
   tatggatcga tcattgcaca cttagtcatg gtccagatgg ccttattgat gtcacgttgg 900
   gtagcacagc cgttaccatt tccaattgca aatttagcca tcaccaaaaa attctattac 960
   tcggagcaga caattcacat gtagacgata aaaaaatgca tgtcacagta gcattcaaca 1020
   ggttcgcaga agcatgtgat caaagaatgc cacgatgtcg atttggattt ttccaagttg 1080
   ttaacaatga ctacaccagc tggggaacgt acgccattgg tggtagtgcc aatcctacta 1140
   tccttagcca aggcaaccga ttccatgctc cgaatgaccc aatgaagaaa aatgtgttgg 1200
   tgagggctga tgcaccacat acagagtcaa tgaagtggaa ttggagatct gagaaagact 1260
   tgttagaaaa tggagctata tttgtagcat cagggtgcga cccgcatcta accccggaac 1320
   aaaaaagcca tttgattcca gctgaaccag gatcagcagt tcttcaactc accagttgtg 1380
   ctggcacgct caaatgcgtt cctggaaaac cttgttaata gttatcaccc actttttatt 1440
   tttattgtta ttagcatttt ttatacttgt taggattgta gtggaatgag acattgatac 1500
   gtgcattaca agaccaaaaa aaaaaaaaaa aaaaa                            1535
<210> 10
   <211> 11
   <212> PRT
   <213> Artificial Sequence
<220>
   <223> Description of Artificial Sequence: Peptide
<400> 10
   Leu Glu Asn Gly Ala Ile Phe Val Ala Ser Gly
   1               5                   10

## Description

Pollen grains originating from weeds, in particular those belonging to the very extensive plant family *Compositae* or *Asteraceae,* are common allergen sources throughout the world. The three most important wind-pollinated genera in this family are *Ambrosia* (ragweed), *Parthenium* (feverfew), and *Artemisia vulgaris* (mugwort). In late summer and autumn, pollen of mugwort is one of the main causes of allergic reactions in central Europe and parts of Asia (J. Charpin, R. Surinyach, and A. W. Frankland (1974) Atlas of European Allergenic Pollens (Paris: Sandoz)). Approximately 10% of patients suffering from pollinosis are sensitized by mugwort pollen. Ragweed pollen represents the major source of allergenic proteins in the United States and Canada, with a prevalence of about 50% in atopic individuals (L. P. Boulet, H. Turcotte, C. Laprise, C. Lavertu, P. M. Bedard, A. Lavoie, and J. Hebert (1997) Comparative degree and type of sensitization to common indoor and outdoor allergens in subjects with allergic rhinitis and/or asthma. Clin Exp Allergy 27, 52). In Europe, ragweed allergy is not a major cause of pollinosis, but it is rapidly increasing. Ragweed was first introduced into Europe at the beginning of last century and was limited to Hungary. During the Second World War it was introduced in France, thereafter gradually expanded and is now abundant in the Rhone valley (France), northern Italy, eastern parts of Austria, Hungary and Bulgaria (G. D'Amato, F. T. Spieksma, G. Liccardi, S. Jager, M. Russo, K. Kontou-Fili, H. Nikkels, B. Wuthrich, and S. Bonini (1998) Pollen-related allergy in Europe. Allergy 53, 567)..

Nowadays, it is widely accepted that recombinant allergens represent promising tools for diagnosis and therapy of Type I allergy. The value of these molecules for diagnosis has been evaluated in detail and a panel of recombinant allergens is already available for routine diagnosis of certain inhalant allergies, including tree (birch)- and grass- pollen allergies (R. Valenta et al. (1992) Int Arch Allergy Immunol 97, 287; O. Scheiner, and D. Kraft (1995) Allergy 50, 384; D. Kraft et al. (1998) Allergy 53, 62; D. Kraft et al. (1999) Int Arch Allergy Immunol 118, 171) but not for weed pollen-allergy. Two major allergenic compounds of ragweed pollen known as antigens E (Amb a 1) and K (Amb a 2) were characterized and shown to be acidic proteins of about 38,000 Da (T. P. King (1980) Allergy 35, 187). cDNA cloning of Amb a 1 and Amb a 2 revealed that these allergens are highly homologous to each other and belong to the family of pectate lyase proteins (T. Rafnar et al. (1991) J Biol Chem 266, 1229; B. L. Rogers et al. (1991) J Immunol 147, 2547). Presently, no information about the complete structure and no molecular cloning data of any mugwort pollen allergen have been published. The only exception is a mugwort pollen allergen designated Art v 1 which is disclosed in WO99/49045. Art v 1 is not related to Amb a 1 or to any other characterized ragweed allergen. Several reports demonstrated that ragweed and mugwort pollen share common allergenic structures, i.e. antigens recognized by cross-reactive lgE antibodies in different allergen sources (C. Fernandez et al. (1993) J Allergy Clin Immunol 92, 660; R. Hirschwehr et al. (1998) J Allergy Clin Immunol 101, 196; H. S. Park et al. (1994) J Korean Med Sci 9, 213). lgE recognition of such common antigens will trigger allergic reactions in sensitized patients after contact with different allergen sources. Therefore, the characterization of mugwort pollen allergens and identification of common allergenic structures in ragweed pollen will provide basic information for the selection and production of recombinant allergens that are sufficient for the diagnosis and therapy of weed pollen-allergy.

WO 96/13589 discloses peptides which comprise at least one T cell epitope selected from the allergens Amb a I.1, Amb a I.2, Amb a I.3, Amb a I.4 and Amb a II. The source of the antigen is Ambrosia artemisilfolia which is the major cause of late summer hayfever in North America and Canada.

Ferreira et al., International Arches of Allergy and Immunology, p. 77-79 (2001) report the cloning of putative mugwort pollen allergens using a panel of 12 monoclonal antibodies raised against mugwort pollen proteins. The publication discloses, however, no sequence of any protein.

Hirschwehr et al., Journal of Allergy and Clinical Immunology ... (1998), p. 1,96-206 describe immunoblotting and inhibition experiments in order to investigate cross-reactive allergens of mugwort and ragweed pollen.

It is therefore an object of the present invention to identify an allergen from mugwort pollen which might trigger type I allergy.

The inventors succeeded in isolating four *bona fide* and complete cDNA clones corresponding to a 40.9 kDa mugwort *(Artemisia vulgaris)* pollen allergen. The amino acid sequence of the novel 40.9 kDa mugwort pollen allergen is shown in SEQ ID NO:1.

The invention relates to an allergen consisting of a polypeptide comprising a fragment of at least 18 amino acids of the amino acid sequence as shown in SEQ 10 NO:1. Such polypeptide comprises at least 18 consecutive amino acids of the sequence as shown in SEQ ID NO:1. Preferably, the length of the fragment is at least 21 amino acids, more preferably at least 25 amino acids, even more preferably at least 35 amino acids, most preferably at least 50 amino acids of the amino acid sequence as shown in SEQ ID NO:1.

Preferably, the allergen consists of a polypeptide whereby the length of said polypeptide is at least 25 amino acids, most preferably at least 35 amino acids. In further embodiments, the polypeptide of the invention consists of at least 18 or at least 21 or at least 30 contiguous amino acids from the amino acid sequence as shown in SEQ ID NO: 1 . In further aspects, the length of the polypeptide is not greater than 100 or 50 or 30 amino acids. Polypeptides consisting of at least 7 amino acids can be recognized by T cells I(T cell epitopes).

Yet another aspect of the invention is an allergen consisting of polypeptide comprising a fragment of the amino acid sequence as shown in SEQ ID NO:1 wherein said fragment is capable of binding to lgE antibodies from an individual being allergic against mugwort pollen. The term "lgE antibodies" means an antibody preparation which is obtainable by per se known processes. In susceptible humans, exposure to allergens leads to an immediate type (IgE-mediated) of allergic response, which is a two-step process:
(1) On the first exposure, allergenic proteins induce lgE synthesis by B-cells (Vercelli and Geha, 1989, J. Allergy Clin. Immunol. 9: 75-83). These specific lgE antibodies then bind to the surfaces of mast cells and basophils via high-affinity Fcε receptors (FcεRl).
(2) On subsequent exposure, allergens bind and crosslink these specific lgE antibodies leading to the release of pre-formed and newly synthesized inflammatory mediators (e.g. histamine) and chemotactic substances (e.g. platelet-activating factor).

In step (1), the production of allergen-specific IgE by B lymphocytes requires the "help" of T lymphocytes (Vercelli and Geha, 1989, J. Allergy Clin. Immunol. 9: 75-83), which are activated by linear peptide fragments of the allergen. These peptides are created by antigen-presenting cells through antigen processing and are displayed at the cell surface with molecules of the major histocompatibility complex (MHC), where they become available for recognition and binding to the T cell receptor (Schwartz, 1985, Annu Rev. Immunol. 3: 237-255; Rothbart et al., 1989, Int. Immunol. 1: 479-486). In step (2), allergen-specific lgE antibodies produced by B cells circulate and bind to FcεRI receptors on mast cells and basophils, thereby serving as the receptor for the allergen. Cross-linking of these cell surface-bound IgE antibodies by allergen represents the signal for the release of preformed and newly synthesized inflammatory mediators and chemotactic substances, leading to the typical allergic inflammatory reaction.

An individual being allergic against mugwort pollen is an individual that displays specific IgE antibodies recognizing mugwort pollen proteins and have typical allergic symptoms (IgE-mediated) when exposed to mugwort pollen. Such allergic reactions can also be triggered by exposure to homologous antigens present in other allergen sources. This phenomenon is defined as cross-reactivity. The selection of patients allergic to mugwort pollen was done according to typical case history, positive skin prick test and radioallergosorbent test "RAST" >3.5.

A polypeptide is capable of binding to an antibody if its affinity to the antibody is significantly higher than that of a reference composition which does not bind to the antibody. The binding of specific IgE antibodies to a particular polypeptide or allergen can be determined by various methods, e.g. RAST, immunoblot, ELISA, using as reference sera from healthy non-allergic individuals (according to case history, negative skin prick test, negative RAST), who do not display allergen-specific IgE antibodies.

In one embodiment of the invention the polypeptide is capable of binding to IgE antibodies from an individual being allergic against ragweed pollen. The present invention makes it possible to identify allergenic structures which are common to mugwort pollen and ragweed pollen. This information is useful for the selection and production of recombinant allergens that are sufficient for the diagnosis and therapy of weed pollen-allergy. Peptides and polypeptides containing such common allergenic structures are encompassed by the present invention.

During purification of natural Amb a 1 trypsin-like pollen protease cleaves this allergen. Thus, purification often results in the isolation of a full-length 38 kDa allergen plus two fragments of 26 and 12 kDa molecular weight, designated alpha (amino acids 187 to 396, numbering including signal peptide) and beta (amino acids 43 to 180, numbering including signal peptide), respectively. All chains are still highly reactive with polyclonal rabbit antibodies and human IgE from allergic patients. It is expected that proteolytic fragments of the natural 40.9 kDa mugwort allergen corresponding to these chains would also bind IgE antibodies from allergic patients. The first one corresponding to beta chain spans amino acids 21 to 180 and the second one corresponding to alpha chain spans amino acids 181 to 396, both numberings referring to SEQ ID NO:1. Both chains are expected to have epitopes which are also present in the Amb a 1 chains resulting in the same lgE reactivity. The invention relates to a polypeptide comprising a fragment of the sequence as shown in SEQ ID NO:1 wherein said fragment consists of amino acids 21 to 180 or 181 to 396 of the sequence as shown in SEQ ID NO:1.

in one embodiment, the polypeptide of the invention is not a polypeptide consisting of the amino acid sequence LENGAIFVASG (SEQ ID NO:10).

According to a preferred embodiment, the polypeptides are isolated polypeptides, i.e. they are in an essentially pure form- "Essentially pure" means that by separation of the polypeptides from other compounds the polypeptides are at least 75% pure, preferably at least 90 % pure, more preferably at least 95% pure. The purity of polypeptides can be determined by the skilled person using techniques known in the field, e.g. by SDS-PAGE followed by protein staining. High Performance Liquid Chromatography (HPLC) and mass spectrometry are methods suitable for analyzing polypeptides and short peptides as well.

The polypeptides can be prepared in various ways. The polypeptides may be prepared by chemical synthesis, preferably by applying solid phase methods. Methods of chemical peptide synthesis are known to the skilled person (see, e.g., Merrifield, J. Am. Chem. Soc. 85:2149, 1963; Stewart et al., "Solid Phase Peptide Sythesis" (second edition), Pearce Chemical Co., Rockford, IL, 1984 and Bayer and Rapp Chem. Pept. Prot. 3:3 (1986); and Atherton et al., Solid Phase Peptide Synthesis: A Practical Approach, IRL Press, Oxford, 1989). These methods are particularly suited for the preparation of short polypeptides having a length of 7 to 50 amino acids. Longer polypeptides may be prepared by chemically or enzymatically linking peptide fragments which have been prepared by chemical synthesis. The polypeptides of the invention may also be prepared by expression of DNA sequences in host cells. These methods are described in more detail below.

The present invention also concerns various polynucleotides. The polynucleotides may be single or double stranded DNA molecules, RNA molecules or nucleic acids which can be derived therefrom. According to a first aspect, the polynucleotides of the invention encode the amino acid sequence as shown in SEQ ID NO:1. Due to the degeneracy of the genetic code, many different polynucleotides can be envisaged which encode the amino acid sequence as shown in SEQ ID NO:1.
According to a second aspect, the polynucleotides of the invention encode a polypeptide according to the invention as described supra.

The polynucleotides described herein can be used to identify similar sequences in any variety or type of mugwort and, thus, to identify or isolate sequences which have sufficient homology to hybridize to, for example, DNA from mugwort pollen. This can be carried out, for example, under conditions of low stringency; those sequences which have sufficient homology (generally greater than 40%) can be selected for further assessment. Alternatively, high stringency conditions can be used. In general, high stringency conditions are selected to be about 5°C to about 10°C lower than the thermal melting point (T_{M}) for the specific sequence at a defined ionic strength and pH. The T_{M} is the temperature (under defined ionic strength and pH) at which 50% of the target sequence hybridizes to a perfectly matched probe. Typical high stringency conditions are those in which the salt concentration is up to about 15 mM Na⁻ (0.1 x SSC) at pH 7, and the temperature is at least about 60°C.

In this manner, DNA of the present invention can be used to identify, in other types of weed pollen sequences encoding peptides having amino acid sequences similar to that of the 40.9 kDa protein described herein and, thus, to identify allergens in such other types of weed pollen. Thus, the present invention discloses not only the 40.9 kDa protein and other mugwort allergens encoded by the present DNA sequences, but also other weed pollen allergens encoded by DNA which hybridizes to DNA of the present invention.

In one embodiment the polynucleotide comprises the nucleotide sequence as shown in SEQ ID NO:2 or the nucleotide sequence as shown in SEQ ID NO:3. In another embodiment, the polynucleotide of the invention comprises at least 100, preferably at least 300, more preferably at least 600, most preferably at least 1000 contiguous nucleotides of the sequence as shown in SEQ ID NO:2 or SEQ ID NO:3.

The invention also covers polynucleotides which are degenerate to the polynucleotides described supra. The respective complementary strands of the polynucleotides disclosed are encompassed by the present invention.

The polynucleotides of the present invention preferably are isolated polynucleotides. The polynucleotides preferably are essentially pure, i.e. they are at least 80 % pure, more preferably at least 90 % pure, most preferably at least 95 % pure. The polynucleotides according to the invention can be prepared in various ways (see, e.g., Glick and Pasternack, Molecular Biotechnology, Principles and Applications of recombinant DNA, ASM Press, Washington D.C. 1994; Itakura et al. Annu. Rev. Biochem. 53:323-56, 1984 and Climie et al., Proc. Natl. Acad. Sci. USA 87:633-7, 1990). They may be synthesized by chemical methods usually employed in oligonucleotide synthesis. PCR based methods can be used to synthesize the polynucleotides, in particular longer polynucleotides (see, e.g. Lee et al., Nucleic Acid Amplification Technologies, Eaton Publishing, Natick, MA 1997; McPherson, M. J. et al. PCR - A Practical Approach, Vol. 1 and 2, IRL Press, Oxford 1996). Longer polynucleotides can also be prepared by chemically or enzymatically linking fragments which have been synthesized using chemical methods.

Another aspect of the invention is a plasmid or a vector comprising a polynucleotide according to the invention. The plasmids may contain regulatory sequences which facilitate replication of the plasmids or transcription and/or translation of encoded sequences. Examples of such sequences are promotors, terminator sequences, etc. The plasmids may also contain nucleotide sequences encoding amino acid sequences which facilitate the purification of encoded polypeptides upon expression in a host cell. Examples of such sequences are a 6xHis tag, a FLAG tag, and sequences encoding bacterial proteins such as GST. Purification can be achieved by affinity chromatography using antibodies directed against the respective tag sequences or bacterial sequences. Plasmids and vectors containing such tag sequences or bacterial sequences are known to the skilled person.

The invention further pertains to a cell containing a plasmid or a vector and a polynucleotide according to the present invention. The cells may be selected from plant cells, bacterial cells, yeast cells and other cells. Preferred are cells which allow for the expression of the genes encoded by the polynucleotides of the invention. Most preferably, the cells are E. coli cells. The plasmid, vector and/or polynucleotide according to the present invention may be introduced into the cells by techniques known per se, e.g. transformation, transfection etc. The cells can contain the nucleic acid molecules only transiently or stably integrated into their genome. In particular in the latter case, the nucleic acid molecules may be truncated or fragmented due to the process of integration into the genome. Cells containing such truncated or fragmented versions of the nucleic acid molecules are within the scope of the present invention.

The cells can be used for the expression and purification of the polypeptides of the invention. The invention therefore relates to a process for the preparation of a polypeptide according to the invention comprising the step of culturing the cells described in the present invention under conditions appropriate for the expression of the polypeptide and optionally subsequently recovering the polypeptide. The cells are preferably cultured in a liquid medium under agitation. If appropriate, gene expression is induced by an inducing agent, e.g. IPTG. Techniques for manipulating cloned DNA molecules and introducing exogenous DNA into a variety of host cells, techniques for transforming these hosts and techniques for expressing foreign DNA sequences cloned therein are well known in the art and disclosed in, e.g., SambrooK et al., Molecular Cloning: A Laboratory Manual, second edition, Cold Spring Harbour Laboratory Press, Cold Spring Harbour, New York 1989; and Ausubel et al., eds., Current Protocols in Molecular Biology, John Wiley and Sons, Inc., New York 1987.

Following cultivation the cells may be opened using established methods and the polypeptide may be recovered by affinity chromatography. Other known methods of protein purification can be employed. Exemplary purification steps may include hydroxyapatite, size exclusion, FPLC and reverse-phase high performance liquid chromatography. Suitable chromatographic media include derivatized dextrans, agarose, cellulose, polyacrylamide, specialty silicas, and the like. In a preferred embodiment, the polypeptide of the invention is isolated by a method comprising an affinity chromatography step. According to this embodiment, one or more antibodies directed against the polypeptide of the invention may be immobilized to a solid support. Preferably, the antibody specifically recognizes the polypeptide of the invention. Methods for binding antibody molecules to support media are well known in the art. Selection of a particular method is a matter of routine design and is determined in part by the properties of the chosen support.

Alternatively, immobilized metal ion adsorption chromatography can be used to purify histidine-rich proteins, including those comprising polyhistidine tags. Briefly, a gel is first charged with divalent metal ions to form a chelate. Histidine-rich proteins will be adsorbed to this matrix with differing affinities, depending on the metal ions used, and will be eluted by competitive elusion, lowering the pH, or use of strong chelating agents. Within additional embodiments of the invention, a fusion of the polypeptide of interest and an affinity tag may be constructed to facilitate purification. Fusion proteins can be prepared by methods known to those skilled in the art by preparing each component of the fusion protein and chemically conjugating them. Alternatively, a polynucleotide encoding both components of the fusion protein in the proper reading frame can be generated using known techniques and expressed by the methods described herein. Methods of protein purification are described, e.g., in Deutscher, M. P. Protein Purification, Academic Press, New York 1990; Scopes, R. K. Protein Purification, Springer Verlag, Heidelberg 1994; Doonan, S. Protein Purification Protocols. Humana Press, 1996.

Polypeptides can be used, for example, as "purified" allergens. Such purified allergens are useful in the standardization of allergen extracts which are key reagents for the diagnosis and treatment of ragweed allergy. Furthermore, by using peptides based on fragments of the amino acid sequence as shown in SEQ ID NO:1, anti-peptide antisera or monoclonal antibodies can be made using standard methods. These sera or monoclonal antibodies, directed against the 40.9 kDa protein of the invention, can be used to standardize allergen extracts.

An antibody which is capable of binding to a polypeptide can be produced. The antibody may be a polyclonal or a monoclonal antibody, monoclonal antibodies, however, are preferred. Antibodies directed against the polypeptide of the invention may be prepared in a known manner, see, e.g., Harlow and Lane 1988 "Antibodies: A Laboratory Manual"; Cold Spring Harbour Laboratory. In a particular embodiment the antibody is in an essentially pure form, i.e. at least 80% pure, preferably at least 90% pure. In the case of polyclonal antibodies, the antibody composition contains many different species of antibody molecules.

The antibody may be specific for the polypeptide comprising the amino acid sequence as shown in SEQ ID NO.1. Such an antibody does not bind to anyone of the polypeptides Amb a 1.1, Amb a 1.2, Amb a 1.3 and Amb a 2 from ragweed pollen.

However, another antibody is capable of binding to one or several (e.g., two, three or four) of the polypeptides Amb a 1.1, Amb a 1.2, Amb a 1.3 and Amb a 2 from ragweed pollen.

Antibodies can be used to isolate additional components of mugwort allergens which can be used for further definition of the characteristics of this family of allergens. Furthermore, anti-peptide sera or monoclonal antibodies directed against the polypeptides of the invention can be used to standardize and define the content of skin test reagents.

The invention further concerns a pharmaceutical composition comprising a polypeptide and/or a polynucleotide according to the present invention.

The materials described herein, as well as compositions containing these materials, can be used in methods of diagnosing, treating and preventing mugwort allergy. Another aspect of the invention therefore is the use of a polypeptide and/or a polynucleotide and/or an antibody according to the present invention for the preparation of a medicament for the treatment or the prevention or the diagnosis of an allergic disorder.

The medicament is preferably administered to an individual to be desensitized.

Through use of the polypeptides of the present invention, allergen preparations of consistent, well-defined composition and biological activity can be made and administered for therapeutic purposes (e.g., to modify the allergic response of a mugwort-sensitive individual to a mugwort pollen). Such polypeptides or modified versions thereof may, for example, modify B-cell response to a mugwort allergen, T-cell response to a mugwort allergen or both responses. Purified allergens can also be used to design modified derivatives or analogues which are more useful in immunotherapy than are the unmodified naturally-occurring peptides.

High doses of allergens generally produce the best symptom relief. However, many people do not tolerate high doses of allergens because of allergic reactions to the allergens. Modification of naturally-occurring allergens can be designed in such a manner that modified polypeptides which have the same or enhanced therapeutic properties as the corresponding naturally-occurring allergen but have reduced side effects, especially reduced anaphylactic reactions, can be produced. These can be, for example, a polypeptide of the present invention or a modified analogue (e.g., a polypeptide in which the amino acid sequence has been altered to modify immunogenicity and/or reduce allergenicity or to which a component has been added for the same purpose). For example, the polypeptides can be modified using the polyethylene glycol method of A. Sehon and co-workers. Short segment deletion or amino acid substitutions of the polypeptide sequence results in a weakened (weaker or no) antibody binding. This modification leads to low IgE binding and is very useful in immunotherapy due to less side effects.

Administration of the peptides of the present invention to an individual to be desensitized can be carried out using known techniques. A peptide or combination of different peptides can be administered to an individual in a composition which includes, for example, an appropriate buffer, a carrier and/or an adjuvant. Such compositions will generally be administered by injection, oral administration, inhalation, transdermal application or rectal administration. Using the structural information now available, it is possible to design a mugwort pollen polypeptide which, when administered to a mugwort-sensitive individual in sufficient quantities, will modify the individual's allergic response to a mugwort allergen. This can be done, for example, by examining the structures of the mugwort proteins, producing peptides to be examined for their ability to influence B-rell and/or T-cell responses in mugwort-sensitive individuals and selecting appropriate epitopes recognized by the cells. Synthetic amino acid sequences which mimic those of the epitopes and which are capable of down regulating allergic response to mugwort allergen can also be used.

Polypeptides or antibodies can also be used for detecting and diagnosing mugwort allergy. For example, by combining blood or blood products obtained from an individual to be assessed for sensitivity to mugwort allergen with an isolated allergenic peptide of mugwort pollen, under conditions appropriate for binding of components (e.g., antibodies, T cells, B cells) in the blood with the polypeptide and determining the extent to which such binding occurs.

It is now also possible to design an agent or a drug capable of blocking or inhibiting the ability of mugwort allergens to induce an allergic reaction in mugwort-sensitive individuals. Such agents could be designed, for example, in such a manner that they would bind to relevant anti-mugwort lgEs, thus preventing lgE-allergen binding and subsequent mast cell degranulation. Alternatively, such agents could bind to cellular components of the immune system, resulting in suppression or desensitization of the allergic response to mugwort allergens. A non-restrictive example of this is the use of appropriate B- and T-cell epitope peptides, or modifications thereof, based on the cDNA/polypeptide structures of the present invention to suppress the allergic response to mugwort allergens. This can be carried out by defining the structures of B- and T-cell epitope peptides which affect B- and T-cell function in in vitro studies with blood cells from mugwort-sensitive individuals.

Finally, the invention relates to kits which are useful for the diagnosis, the treatment and/or the prevention of an allergic disorder comprising a polypeptide and/or a polynucleotide. The materials described herein, as well as compositions and kits containing these materials, can be used in methods of diagnosing, treating and preventing mugwort allergy.

The present invention is based on a recombinant DNA molecule and fragments thereof coding for a 40.9 kDa mugwort *(Artemisia vulgaris)* pollen allergen, which shows sequence homology to Amb a 1, a major allergen of ragweed *(Ambrosia artemisiifolia)* pollen, on a method for the isolation of Art v 1 molecules or fragments thereof, and on an expression vector and a host cell transformed to express the nucleic acid sequences of the invention. The present invention further relates to methods of diagnostics and therapy of weed pollen-allergic patients. Because the 40.9 kDa mugwort allergen shows sequence homology to Amb a 1, this mugwort protein might represent a cross-reactive allergen and could be used for diagnosis and therapy of not only mugwort pollen allergy, but of weed pollen-allergy in general.

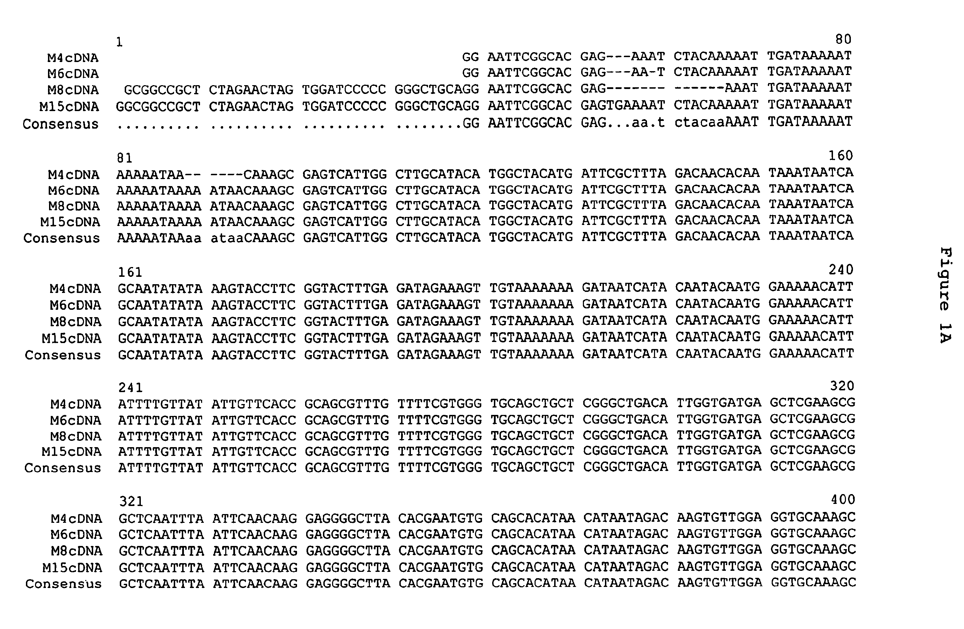

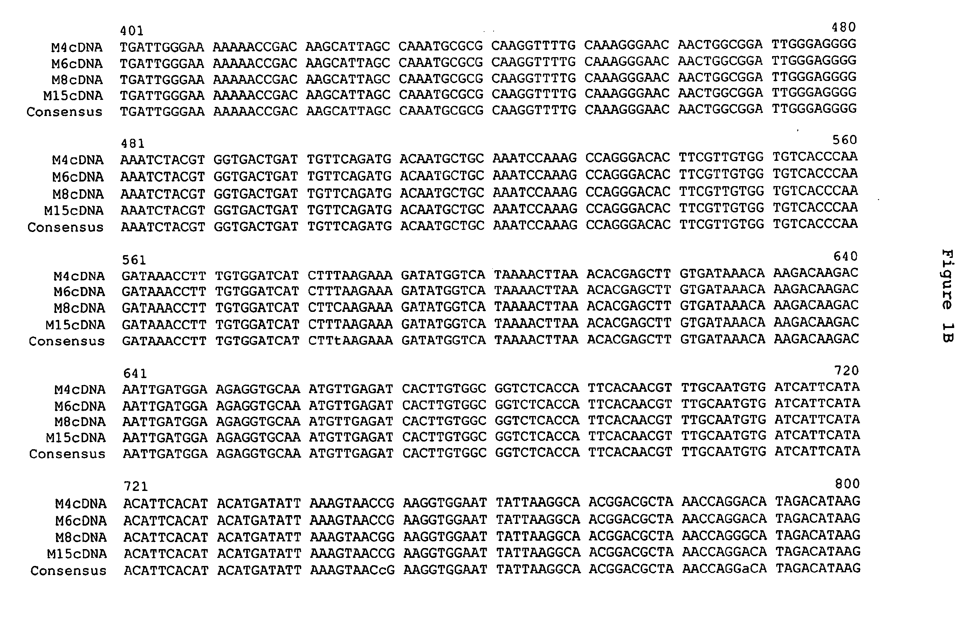

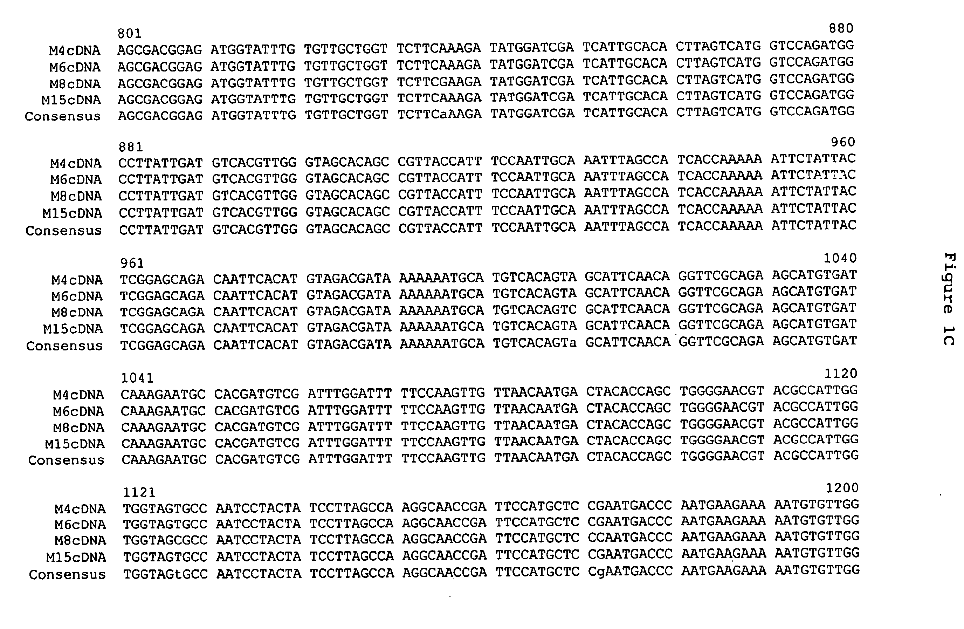

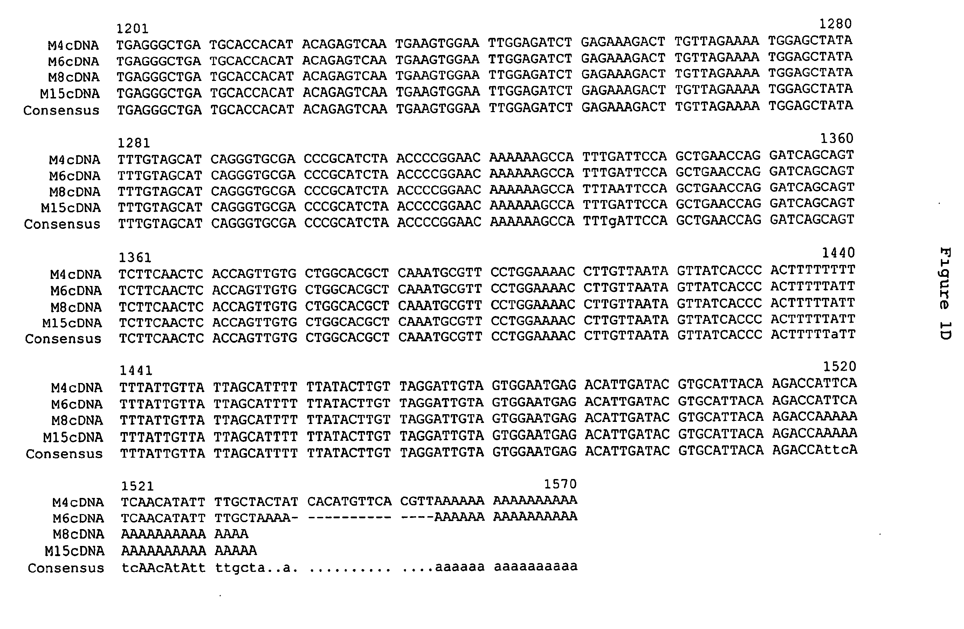

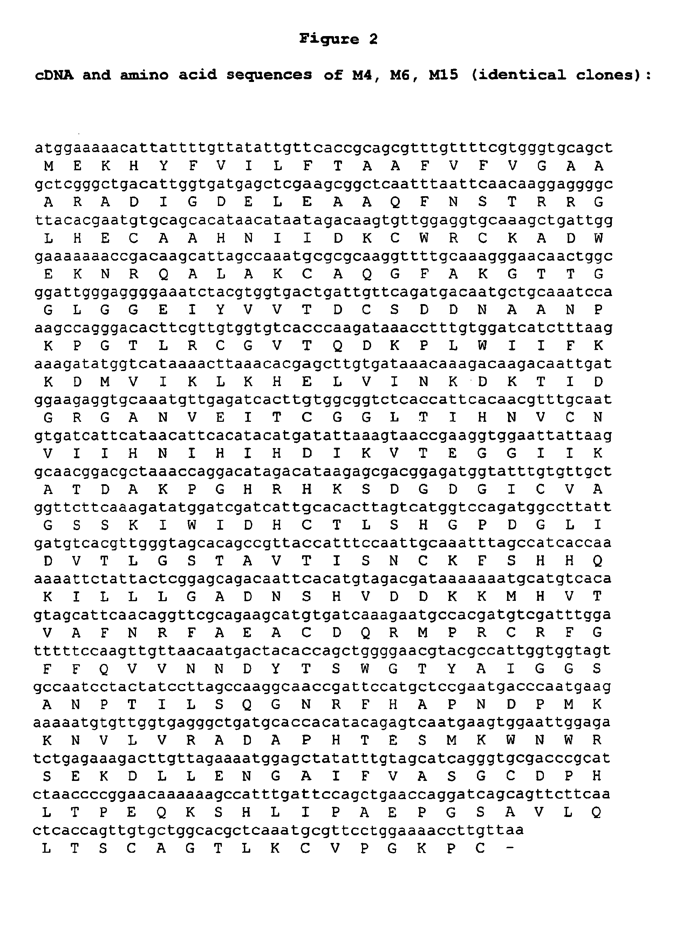

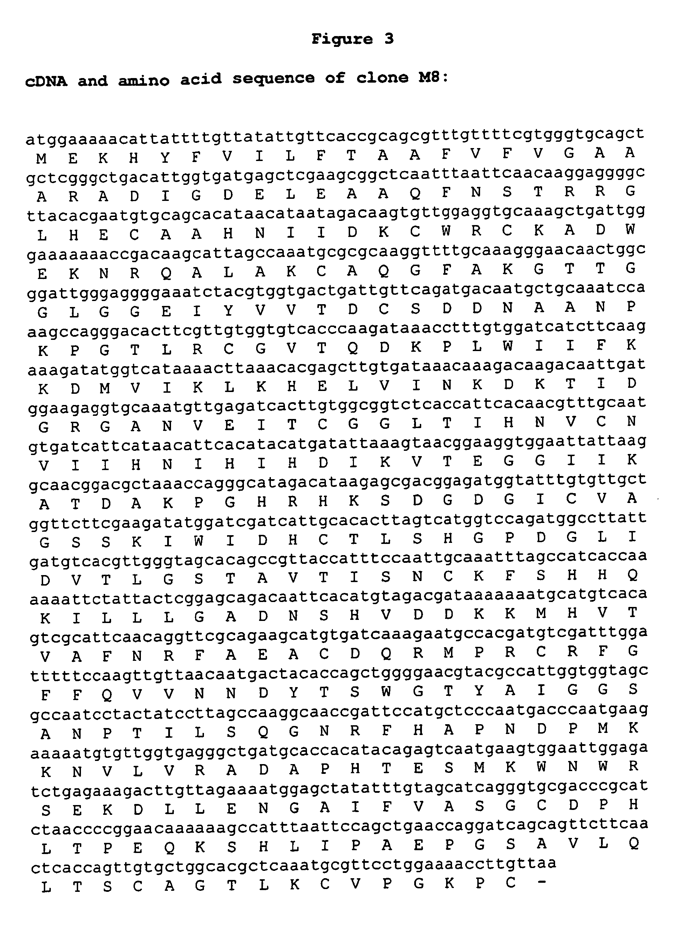

The present application describes the isolation of four *bona fide* and complete cDNA clones corresponding to a 40.9 kDa mugwort (Artemisia vulgaris) pollen allergen. The cDNA nucleotide sequences and derived amino acid sequences of the clones M4, M6, and M15 were identical (See Figures 1 and 2). Clone M8 differed from the other by 8 nucleotide substitutions, which did not lead to amino acid exchanges (See Figure 3). The clones are complete in their 5' ends because they contain the start AUG codon in a typical eukaryotic context and some sequence upstream of the start codon. The clones are complete in the 3' region because they contain 90-120 nucleotides after the stop codon followed by the polyA⁺-tail. The sequences outside the open reading frame are not shown in Figures 2 and 3. The alignments of the nucleotide sequences of clones M4, M6, M8 and M15 are shown in Figure 1A-D. The nucleotide sequence of the coding region and the deduced amino acid sequence of clones M4, M6 and M15 are shown in Figure 2. The nucleotide sequence of the coding region and the deduced amino acid sequence of clone M8 are shown in Figure 3.
Figures 1A, 1B, 1C and 1D show an alignment of the nucleotide sequences of clones M4, M6, M8 and M15 (SEQ ID NO:6, 7, 8, and 9). Capitals in the consensus sequence indicate identical nucleotides in the four clones.
Figure 2 shows the nucleotide sequence of the coding region (SEQ ID NO:2 plus stop codon) and the deduced amino acid sequence (SEQ ID NO:1) of clones M4, M6 and M15.
Figure 3 shows the nucleotide sequence of the coding region (SEQ ID NO:3 plus stop codon) and the deduced amino acid sequence (SEQ ID NO:1) of clone M8.
Figure 4 shows the structure of the multiple cloning site of plasmid "pHis-Parallel2" as used in Example 2. The amino acids encoding a 6xHis tag, a spacer region and a protease cleavage site are also indicated.
Figure 5A shows Mugwort pollen extract, purified mugwort pollen allergen and purified Amb a 1 from ragweed pollen separated by gel electrophoresis followed by Coomassie staining. Figure 5B shows an immunoblot with rabbit anti-Amb a 1 antibodies (See Examples 3 and 4).
Figure 6 shows an immunoblot with IgE antibodies (See Examples 3 and 4).
Figure 7 shows a Coomassie-stained gel after SDS-PAGE. Lane 1 (left): Molecular weight marker RPN 756 (Amersham Biosciences); lane 2 (middle): Mugwort pollen extract; lane 3 (right): purified recombinant 40.9 kDa mugwort pollen protein, ~ 3 µg (see Example 5).

The present invention will be further illustrated by the following examples which are not intended to be limiting.

### Example 1: Immunoscreening of a Mugwort Pollen cDNA Library with Purified Rabbit anti-Amba 1 Antibodies

The isolation of the cDNA clone coding for the 40.9 kDa mugwort pollen allergen was clone in the following way:

The first step for the cDNA cloning of the 40.9 kDa allergen was the isolation of RNA from mugwort pollen, which turned out to be a very difficult procedure. Using several different standard procedures for RNA isolation, there were always pigments and other organic substances co-purifying with the RNA that inhibited enzymes used for the cDNA synthesis. However, after a series of trials it was possible to isolate mRNA sufficiently pure to construct a cDNA library, which was also clone in the expression vector lambda ZAP.

Sera from rabbits immunized with Amb a 1 was kindly provided by Dr. Te Piao King, (Rockefeller University, USA). For screening the mugwort cDNA library we first purified Amb a 1-specific antibodies by affinity chromatography. For that purpose, 5 mg of Amb a 1 purified from ragweed pollen was coupled to CNBr-activated Sepharose (Pharmacia). After binding of the rabbit sera, the resin was washed and Amb a 1-specific antibodies eluted with 0.2 M glycine, pH 2.8. The antibodies were neutralized and dialyzed against PBS. With these purified rabbit anti-Amb a 1-antibodies 450.000 plaques of a mugwort pollen cDNA library constructed in lambda ZAP II phages (Stratagene, CA, USA) were screened. In total, 13 positive clones were isolated and used for single clone *in vivo excision* of pBluescript phagemid from the Uni- ZAP XR vector. Four clones (M4, M6, M8, and M15) were selected for DNA sequence analysis, which was carried out by the "primer walking" technique using 5 primers including the flanking primers T7 und T3. Both strands were sequenced twice. The aligned nucleotide sequences of the four clones M4, M6, M8, and M15 are shown in Figures 1A-1D. The cDNA sequences of clones M4, M6 and M15 were identical (Figure 2). The cDNA sequence in the coding region of M8 differed from the other 3 clones by 8 bases (Figure 3). However, all four clones lead to the same protein with a calculated molecular weight of 40.9 kDa (Figures 2 and 3).

Finally, the sequence of the 40.9 kDa protein was used for similarity searches in the Database. Significant sequence homology was found to Amb a 1, a major allergen from ragweed pollen belonging to the pectate lyase family (63.9 % identity).

The cDNA of the invention can now be obtained based on the sequence information as described herein. The cDNA can be cloned from a cDNA library using as a probe an oligonucleotide or polynucleotide which was prepared by chemical synthesis. Alternatively, the cDNA can be obtained by PCR using primers which are derived from SEQ ID NO:2. Such methods are known to the skilled person.

### Example 2: Cloning of the 40.9 kDa mugwort allergen (clone M4) into the expression plasmid pHIS- parallel-2

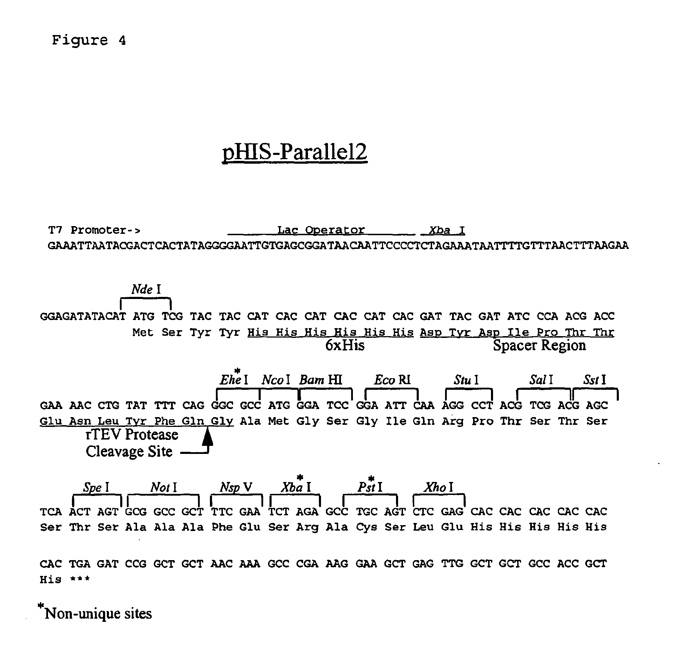

The expression plasmid containing the 40.9 kDa mugwort allergen cDNA was constructed in the vector pHis-parallel 2 (see Figure 4). For the cloning procedure two flanking cloning primers were constructed. The complete cDNA sequence was truncated at the 5' end by 60 nucleotides coding for the putative signal peptide. In this way only the mature form of the protein was produced in E. coli.

The following primers were used: Mug-Nco-fwd 5' GAG AGA GAC CAT GGC TCG GGC TGA CAT TGG TGA TGA GCT CG 3' (SEQ ID NO:4) and Mug-Xho-rev 5' GAG AGA GAC TCG AGT TAA CAA GGT TTT CCA GGA ACG CAT TTG 3' (SEQ ID NO:5) for PCR, Nco I and Xho I restriction sites were introduced at the 5' and 3' ends. PCR products were digested with Nco l and Xho l restriction enzymes and ligated to the respective sites of the vector pHis-parallel-2. The clone was analyzed by DNA sequencing.

### Example 3: Expression of the recombinant 40.9 kDa mugwort allergen in E. coli

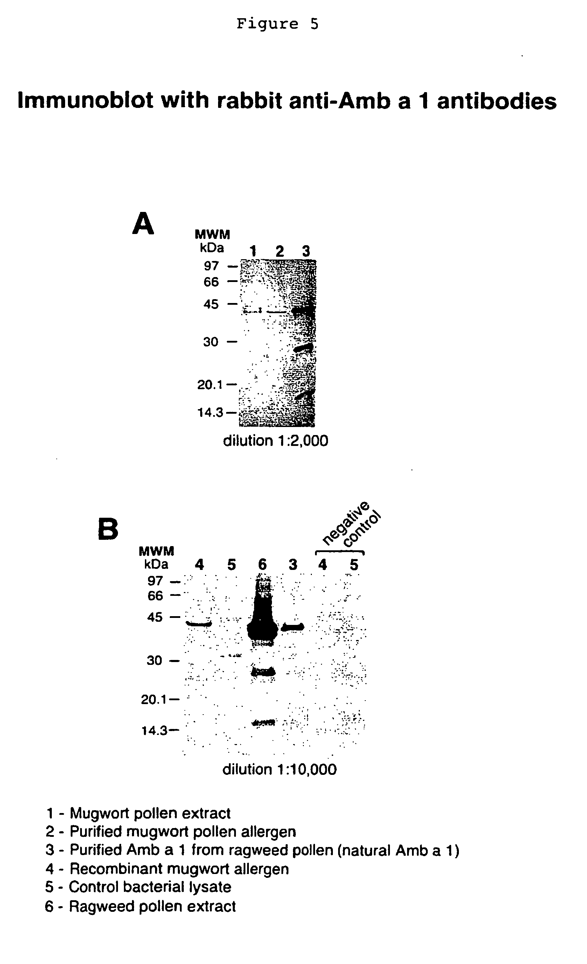

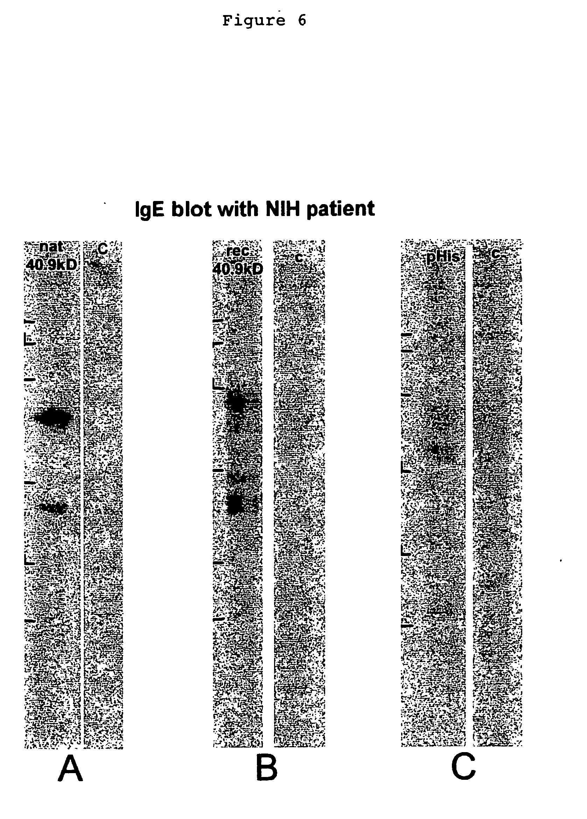

For the recombinant production of the 40.9 kDa mugwort allergen, competent E. coli strain BL21 (DE3, Stratagene, CA, USA) was transformed with the expression plasmid pHis-parallel-2-M4 and selected on LB plates containing 100 mg/l ampicillin. A single transformant colony was picked and bacteria were grown in a 25 ml overnight pre-culture with LB medium containing ampicillin to an optical density of 0.6 at 600 nm. LB-amp medium was added to a volume of 250 ml. Isopropyl-β-D-galactopyranoside (IPTG) was added to a final concentration of 1 mM and incubation continued for 7 hours at 37°C. The bacterial cells were harvested by centrifugation and pellets were resuspended in 50 mM phosphate buffer pH8 containing 300 mM NaCl and 10 mM imidazole. Cells were then disrupted by three cycles of freezing in liquid nitrogen, followed by thawing at 37°C. After centrifugation at 5.500 rpm for 25 minutes, the supernatant was removed (soluble fraction) and 6 M urea was added to the pellet. After centrifugation, the supernatant was removed (insoluble fraction), and both soluble and insoluble fractions analyzed by SDS-PAGE. The recombinant 40.9 kDa mugwort protein was recovered in the insoluble fraction, which was then used for immunoblots with anti-Amb a 1 antibodies (Figure 5B, lane 4) and with serum lgE from weed pollen-allergic patients (Figure 6, lanes 3 and 4). As control, E. coli strain BL21 transformed with pHis-parallel-2 without an insert was processed as described above and the insoluble fraction used for the immunoblot experiments (Figure 6, lanes 5 and 6).

Figure 5A, lane 1 shows the mugwort pollen extract which contains all the proteins (allergens or nonallergens) released from the pollen. Preparation of the mugwort pollen extract:

150 mg of mugwort pollen grains (Allergon AB, Sweden) were mixed with 1 ml of water. The pollen suspension was gently agitated at room temperature for a 10 hour period. After centrifugation the pattern of proteins released from the mugwort pollen was analyzed by SDS-PAGE and Coomassie staining.

Lane 2 of Figure 5A shows purified natural 40.9 kDa mugwort pollen allergen. The purification was clone via immunoaffinity chromatography method. Purified rabbit anti Amb a 1 antibodies were coupled to a cyanogen bromide acivated sepharose CL4B (Pharmacia Biotech) and used for the purification of the natural 40.9 kDa mugwort protein from a filtered 10 hours pollen extract.

Lane 3 of Figure 5A shows purified natural Amb a 1, a major allergen in the ragweed pollen. The purification was also clone via immunoaffinity chromatography method. Purified rabbit anti Amb a 1 antibodies were coupled to a cyanogen bromide acivated sepharose CL4B (Pharmacia Biotech) and used for the purification of the natural Amb a 1 from a filtered 10 hours ragweed pollen extract including 10 mM protease inhibitor 1,10 phenantroline.

### Example 4: Purification of the natural 40.9 kDa allergen from mugwort pollen

The natural 40.9 kDa allergen was purified from aqueous extract of mugwort pollen by immunoaffinity chromatography. Purified rabbit anti-Amb a 1 antibodies were coupled to CNBr-activated sepharose CL4B and used for the purification of the 40.9 kDa allergen. The purified natural 40.9 kDa allergen was then tested in immunoblots with anti-Amb a 1 antibodies (Figure 5A, lane 2) and with lgE antibodies from allergic patients (Figure 6, lanes 1 and 2).

A cross-reactivity is occurring between the US patient sensitized for ragweed and the purified natural and recombinant 40.9 kDa protein in mugwort. Therefore a positive signal can be observed in Figure 6A and 68. It is expected that many more patients show a cross-reactivity between these two allergens.

### Example 5: Expression and Purification of Recombinant 40.9 kDa Mugwort Pollen Protein

The protein expression was carried out in E. coli BL21-DE3 hosting a pHis parallel 2 plasmid with inserted cDNA coding for the 40.9 kDa mugwort pollen protein.
The culture medium (1 % Peptone, 0.5% Yeast Extract, 0.5% NaCl, 100g Glycerin/1L, 2 mM MgSO₄; 20 g (NH₄)₂SO₄, 10 mM PO₄,pH adjusted to 7.4) was inoculated with 3% of a saturated overnight culture, grown in the culture medium with 150 µg/mL penicillin G (Biochemie, Kundl, Austria). Fermentation was carried out in a 10 L Bioflow 3000 Fermenter (New Brunswick Scientific Co., Edison, NJ) at 37°C with 7% oxygen saturation 200 - 400 rpm agitation and induction with 0.4 mM IPTG at an optical density of 0.8.

Bacterial cells were harvested by centrifugation 5 hours post induction (20 g wet cell weight from 10 L culture) and resuspended in 100 mL lysis buffer (50 mM Tris base, 1 mM EDTA, 0.1% Triton X-100, pH non-adjusted, 5 mUg cells). After addition of freshly dissolved lysozyme (100 µg/g cells) and incubation for 1 hour at room temperature, the cells were lysed by 3 freeze-thaw cycles at -70°C and 37°C. The DNA was sheared with an ultraturax. Separation of the soluble fraction was clone by centrifugation and the raw inclusion bodies were washed twice with 1% Triton X-100, 20 mM Tris-HCl pH 8.0, 1 mM EDTA, followed by 2 washes with 50% ethanol, 20 mM Tris-HCl pH 8.0. Purified inclusion bodies were dissolved in 500 mL 8 M urea, 0.5 M NaCl, 20 mM TrisHCl pH 8.0 and loaded on a 150 mL Chelating Cellufine Column (Millipore, Bedford, MA, USA) pre-equilibrated with the same buffer, after charging it with 0,1 M NiCl₂ according to the manufacturers instructions. All chromatography steps were carried out on a Biopilot FPLC system (Amersham Biosciences, Uppsala, Sweden). Bound protein was eluted with a linear imidazole gradient ranging from 0-500 mM. The purity of the fractions was analyzed by conventional SDS-PAGE. Due to impurity (high background) of the fractions containing the recombinant 40.9 kDa mugwort pollen protein, cation exchange chromatography under denaturing conditions was carried out.

A 180 mL column with the CEC matrix "Matrex Cellufine C-500" (Millipore) was equilibrated with 200 mL 100 mM NaAc pH 5.5. After washing the column with 400 mL binding buffer (5 M urea, 20 mM NaAc, 10 mM β-mercaptoethanol, 2 mM EDTA, pH 5.5) the protein solution was applied with a pH of 5.5.

Again, the fractions were analysed by SDS-PAGE and Coomassie staining. The fractions containing the protein of interest were pooled and prepared for the gel filtration step under denaturing conditions and a basic pH.

Gel filtration was performed in 6 M urea, 0.3 M NaCl, 30 mM TrisHCl pH 8.0, 2 mM EDTA, 10 mM β-mercaptoethanol on a Sephacryl S-200 HR (Amersham Biosciences, Uppsala, Sweden) column (dimensions 50 × 1000 mm).
Pure 40.9 kDa mugwort pollen protein fractions were concentrated and refolded by filtration through an Amicon ultrafiltration device (millipore) while adding 5 mM NH₄HCO₃. The overall yield was 4-6 mg protein.

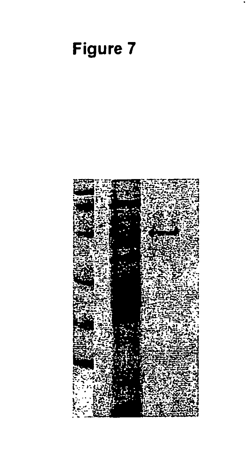

Figure 7, lane 3 shows the purified recombinant 40.9 kDa mugwort pollen allergen with His-tag (~ 3 µg) after SDS-PAGE and Coomassie staining.

### Example 6: IgE Blots with recombinant 40.9 kDa mugwort pollen protein

Sera from different allergic or sensitized patients were tested for reactivity to the novel 40.9 kDa mugwort pollen allergen. The tests were carried out according to the following protocol:
• preparative 15% SDS gels
• load ~ 80 µg purified recombinant 40.9 kDa protein (obtained, e.g., according to Example 5)
• SDS-gel electrophoresis 100V, 2.5 hrs
• Western blot: on a PVDF membrane 2 hrs, 300mA
• Incubation with 1^{st} antibody: human sera: dilute 1:10 in gold buffer for at least 6 hrs at RT
• Wash step 3x 10' in gold buffer
• Incubation with 2^{nd} antibody: Radiolabeled [¹²⁵l]-Anti-Human lgE (also referred as RAST), delivered by MedPro (Cat.# VE39020, 5.2 ml, 5 µCi) - 1:40 in gold buffer, ON at RT
• wash 3x10' in gold buffer
• place the phosphor-imager screen with the white site heading the membrane strips
• expose screen in phosphoimager cassette 24 - 48 hrs
• develop the screen in the phosphor-imager.

| | |
|---|---|
| Blocking Buffer = Gold buffer (1 L): | 7.5 g Na₂HPO₄ |
| | 1.0 g NaH₂PO₄ |
| | 5.0 g BSA |
| | 5.0 ml Tween20 |
| | |
| Transfer buffer (per 5 L): | 15.13 g Tris base |
| | 72.06 g glycine |
| | 1 L methanol absolute |
| | Add dH₂O |

A serum was classified "IgE-positive" when the signal corresponding to the 40.9 kDa band was significantly above the background signal.

The following results were obtained:

**Table 1: Ragweed reference sera from NIH / USA (lane 1 - 3) and mugwort allergic patients from Austria (lane 4 - 22): Blot I**

| **#** | **Patient** | **IgE-positive** | **IgE-positive** |
|---|---|---|---|
| 1 | Human anti ragweed | | x |
| 2 | #3130 | x | |
| 3 | #3131 | | x |
| 4 | 180.212 | | x |
| 5 | 181.822 | | x |
| 6 | 492.788 | | x |
| 7 | 493.050 | x | |
| 8 | 493.087 | | x |
| 9 | 493.201 | | x |
| 10 | 493.021 | | x |
| 11 | 494.190 | | x |
| 12 | 180.912 | | x |
| 13 | 180.889 | x | |
| 14 | 180.682 | x | |
| 15 | 180.178 | x | |
| 16 | 493.647 | | x |
| 17 | 493.173 | x | |
| 18 | 493.12x | | x |
| 19 | 492.712 | | x |
| 20 | 494.102 | | x |
| 21 | 493.910 | | x |
| 22 | 493.885 | x | |

**Table 2: Mugwort allergic patients from Austria (lane 23 - 44): Blot II**

| **#** | **Patient** | **lgE-positive** | **lgE-negative** |
|---|---|---|---|
| 23 | 493.593 | x | |
| 24 | 493.510 | | x |
| 25 | 493.485 | x | |
| 26 | 493.428 | | x |
| 27 | 493.311 | x | |
| 28 | 494.169 | | x |
| 29 | 494.105 | | x |
| 30 | 492.395 | | x |
| 31 | 492.391 | x | |
| 32 | 183.011 | | x |
| 33 | 182.881 | x | |
| 34 | 182.729 | x | |
| 35 | 182.726 | x | |
| 36 | 182.484 | | x |
| 37 | 182.483 | x | |
| 38 | 182.333 | x | |
| 39 | 182.259 | | x |
| 40 | 182.083 | | x |
| 41 | 181.713 | | x |
| 42 | 181.498 | | x |
| 43 | 181.237 | | x |
| 44 | 180.935 | | x |

**Table 3: Ragweed sensitized patients from USA (lane 45 - 58) and Austria (lane 59 - 63): Blot III**

| **#** | **Patient** | **IgE-positive** | **IgE-negative** |
|---|---|---|---|
| 45 | 2 | x | |
| 46 | 3 | x | |
| 47 | 5 | x | |
| 48 | 8 | x | |
| 49 | 14 | x | |
| 50 | 15 | x | |
| 51 | 17 | x | |
| 52 | 18 | x | |
| 53 | 19 | x | |
| 54 | 24 | x | |
| 55 | 25 | x | |
| 56 | 27 | x | |
| 57 | 28 | x | |
| 58 | 29 | x | |
| 59 | 1 | x | |
| 60 | 4 | x | |
| 61 | 7 | x | |
| 62 | 9 | x | |
| 63 | 10 | x | |

**Table 4: Mugwort allergic patients from Salzburg (lane 64 - 68) and Art v 1 T cell patients (lane 69 - 83): Blot IV**

| **#** | **Patient** | **IgE positive** | **IgE-negative** |
|---|---|---|---|
| 64 | 1 | x | |
| 65 | 2 | x | |
| 66 | 3 | x | |
| 67 | 4 | x | |
| 68 | 5 | x | |
| 69 | 1 | x | |
| 70 | 7 | x | |
| 71 | 10 | x | |
| 72 | 14 | x | |
| 73 | 15 | x | |
| 74 | 16 | x | |
| 75 | 22 | x | |
| 76 | 22 | x | |
| 77 | 23 | x | |
| 78 | 24 | x | |
| 79 | 25 | x | |
| 80 | 26 | x | |
| 81 | 27 | x | |
| 82 | 29 | x | |
| 83 | 31 | x | |

**Table 5: Ragweed allergic patients form Canada (lane 84 - 107): Blot V**

| **#** | **Patient** | **IgE-positive** | **IgE-negative** |
|---|---|---|---|
| 84 | C1-6 | x | |
| 85 | C1-8 | x | |
| 86 | C1-11 | | x |
| 87 | C1-12 | x | |
| 88 | C2-17 | x | |
| 89 | C2-18 | | x |
| 90 | C2-19 | | x |
| 91 | C3-25 | | x |
| 92 | C3-27 | | x |
| 93 | C4-33 | x | |
| 94 | C5-36 | | x |
| 95 | C5-38 | x | |
| 96 | C5-40 | | x |
| 97 | C5-43 | | x |
| 98 | C5-48 | | x |
| 99 | C5-52 | | x |
| 100 | C5-55 | | x |
| 101 | C5-56 | x | |
| 102 | C6-59 | | x |
| 103 | C6-61 | | x |

**Table 6: Ragweed allergic patients from Canada and Austria (lane 108 -117): Blot VI**

| **#** | **Patient** | **lgE-positive** | **lg E-negative** |
|---|---|---|---|
| 104 | C6-68 | | x |
| 105 | C6-69 | | x |
| 106 | C11-78 | | x |
| 107 | C11-82 | | x |
| 108 | 5 | | x |
| 109 | 10 | | x |
| 110 | 11 | | x |
| 111 | 12 | | x |
| 112 | 13 | | x |
| 113 | 14 | | x |
| 114 | 15 | | x |
| 115 | 16 | | x |
| 116 | 17 | | x |
| 117 | 18 | | x |

**Table 7: Negative controls: Blot VII (2^{nd} antibody only!!)**

| **#** | **Patient** | **lgE-positive** | **lg E-negative** |
|---|---|---|---|
| 118 | ----- | | x |
| 119 | ----- | | x |
| 120 | ----- | | x |
| 121 | ----- | | x |
| 122 | ----- | | x |
| 123 | ----- | | x |

### Results of the tested patient sera:

Mugwort allergic patients from Austria (total amount of tested patient sera = 61)
IgE - positive: 36 = **59%**
IgE - negative: 25 = **41%**

Ragweed allergic patients from USA, Canada and Austria (total amount of tested patient sera = 56)
IgE - positive: 27 = **48%**
IgE - negative: 29 = **52%**

### Overall results:

Total amount of available sera from mugwort allergic patients: 73
IgE - positive: 36 = **49.4%**
IgE - negative: 37= **50.6%**

Total amount of available sera from ragweed allergic patients: 130
IgE - positive: 27 = **20.8 %**
IgE - negative: 103 = **79.2 %**

Almost 50% of the mugwort sensitized patients and around 20% of the ragweed sensitized patients have lgE antibodies against the 40.9 kDa allergen. This is a relatively high percentage of patients sensitized to this mugwort pollen allergen. Consequently it is important to add this molecule to a vaccine preparation for treatment of these patients' allergy. Novel approaches for allergy therapy go in the direction of patient-tailored vaccine preparations.

## Claims

1. Allergen consisting of a polypeptide comprising a fragment of at least 18 consecutive amino acids of the amino acid sequence as shown in SEQ ID NO:1.

2. Allergen according to claim 1, wherein said fragment is capable of binding to IgE antibodies from an individual being allergic against mugwort pollen.

3. Allergen according to claim 1 or 2, comprising the amino acid sequence as shown in SEQ ID NO:1.

4. Allergen according to any one of claims 1 to 3, **characterized in that** it is capable of binding to IgE antibodies from an individual being allergic against ragweed pollen.

5. A polynucleotide selected from the group consisting of
a) polynucleotides encoding the amino acid sequence as shown in SEQ ID NO:1;
b) polynucleotides encoding a polypeptide as claimed in claim 1 and
c) polynucleotides comprising the nucleotide sequence as shown in SEQ ID NO:2 or the nucleotide sequence as shown in SEQ ID NO:3,
or the complementary strand of such polynucleotide.

6. A plasmid or a vector comprising a polynucleotide as claimed in claim 5.

7. A cell containing a plasmid or a vector as claimed in claim 6.

8. A cell according to claim 7 which is selected from the group consisting of plant cells, bacterial cells and yeast cells.

9. A process for the preparation of an allergen as claimed in any one of claims 1 to 4 comprising the step of culturing cells as claimed in claim 7 or 8 under conditions appropriate for the expression of the polypeptide and optionally subsequently recovering the polypeptide.

10. A process according to claim 9 wherein the cells are opened and the polypeptide is recovered using affinity chromatography.

11. A pharmaceutical composition comprising an allergen as claimed in any one of claims 1 to 4 and/or a polynucleotide as claimed in claim 5.

12. The use of an allergen as claimed in any one of claims 1 to 4 or a polynucleotide as claimed in claim 5 for the preparation of a medicament for the treatment or the prevention of weed pollen allergy.

13. A use according to claim 12 wherein the medicament is administered to an individual to be desensitzed.

14. A kit useful for the diagnosis, the treatment and/or the prevention of an allergic disorder comprising an allergen as claimed in any one of claims 1 to 4 and/or a polynucleotide as claimed in claim 5.

15. The use of an allergen as claimed in any one of claims 1 to 4 or a polynucleotide as claimed in claim 5 for the diagnosis of weed pollen allergy.

## Patentansprüche

1. Allergen, bestehend aus einem Polypeptid, das ein Fragment aus mindestens 18 aufeinander folgenden Aminosäuren der Aminosäuresequenz, wie in der SEQ ID NO: 1 gezeigt, umfasst.

2. Allergen nach Anspruch 1, worin das Fragment in der Lage ist, an IgE-Antikörper aus einem Individuum, das auf Beifußpollen allergisch ist, zu binden.

3. Allergen nach Anspruch 1 oder 2, umfassend die Aminosäuresequenz, wie in der SEQ ID NO: 1 gezeigt.

4. Allergen nach einem der Ansprüche 1 bis 3, **dadurch gekennzeichnet, dass** es in der Lage ist, an IgE-Antikörper aus einem Individuum, das auf Traubenkrautpollen allergisch ist, zu binden.

5. Polynukleotid, ausgewählt aus der Gruppe bestehend aus
a) Polynukleotiden, die für die Aminosäuresequenz, wie sie in der SEQ ID NO: 1 gezeigt ist, codieren;
b) Polynukleotiden, die für ein Polypeptid gemäß Anspruch 1 codieren, und
c) Polynukleotiden, die die Nukleotidsequenz, wie in der SEQ ID NO: 2 gezeigt, oder die Nukleotidsequenz, wie in der SEQ ID NO: 3 gezeigt, umfassen
oder der komplementäre Strang eines solchen Polynukleotids.

6. Plasmid oder Vektor, umfassend ein Polynukleotid gemäß Anspruch 5.

7. Zelle, enthaltend ein Plasmid oder einen Vektor gemäß Anspruch 6.

8. Zelle nach Anspruch 7, die ausgewählt ist aus der Gruppe bestehend aus Pflanzenzellen, Bakterienzellen und Hefezellen.

9. Verfahren zur Herstellung eines Allergens gemäß einem der Ansprüche 1 bis 4, umfassend den Schritt des Kultivierens der Zellen gemäß Anspruch 7 oder 8 unter Bedingungen, die für die Expression des Polypeptids und gegebenenfalls des anschließenden Rückgewinnens des Polypeptids geeignet sind.

10. Verfahren nach Anspruch 9, worin die Zellen aufgeschlossen werden und das Polypeptid mittels Affinitätschromatographie rückgewonnen wird.

11. Pharmazeutische Zusammensetzung, umfassend ein Allergen gemäß einem der Ansprüche 1 bis 4 und/oder ein Polynukleotid gemäß Anspruch 5.

12. Verwendung eines Allergens gemäß einem der Ansprüche 1 bis 4 oder eines Polynukleotids gemäß Anspruch 5 zur Herstellung eines Arzneimittels zur Behandlung oder Prophylaxe einer Allergie auf Unkrautpollen.

13. Verwendung nach Anspruch 12, worin das Arzneimittel einem zu desensibilisierenden Individuum verabreicht wird.

14. Kit, das zur Diagnose, Behandlung und/oder Prophylaxe einer allergischen Erkrankung nützlich ist, umfassend ein Allergen gemäß einem der Ansprüche 1 bis 4 und/oder ein Polynukleotid gemäß Anspruch 5.

15. Verwendung eines Allergens gemäß Anspruch 1 bis 4 oder eines Polynukleotids gemäß Anspruch 5 zur Diagnose einer Allergie auf Unkrautpollen.

## Revendications

1. Allergène consistant en un polypeptide comprenant un fragment d'au moins 18 acides aminés consécutifs de la séquence d'acides aminés telle que représentée dans la SEQ ID NO : 1.

2. Allergène selon la revendication 1, dans lequel ledit fragment est capable de se lier à des anticorps IgE provenant d'un individu qui est allergique au pollen d'armoise.

3. Allergène selon la revendication 1 ou 2, comprenant la séquence d'acides aminés telle que représentée dans la SEQ ID NO : 1.

4. Allergène selon l'une quelconque des revendications 1 à 3, **caractérisé en ce qu'**il est capable de se lier à des anticorps IgE provenant d'un individu qui est allergique au pollen d'ambroisie.

5. Polynucléotide choisi dans le groupe consistant en :
a) des polynucléotides codant pour la séquence d'acides aminés telle que représentée dans la SEQ ID NO : 1 ;
b) des polynucléotides codant pour un polypeptide tel que défini dans la revendication 1 et,
c) des polynucléotides comprenant la séquence nucléotidique telle que représentée dans la SEQ ID NO : 2 ou la séquence nucléotidique telle que représentée dans la SEQ ID NO : 3 ;
ou le brin complémentaire d'un tel polynucléotide.

6. Plasmide ou vecteur comprenant un polynucléotide tel que défini dans la revendication 5.

7. Cellule contenant un plasmide ou un vecteur tel que défini dans la revendication 6.

8. Cellule selon la revendication 7 qui est choisie dans le groupe constitué des cellules végétales, des cellules bactériennes et des cellules de levures.

9. Procédé pour la préparation d'un allergène tel que défini dans l'une quelconque des revendications 1 à 4, comprenant l'étape consistant à mettre en culture des cellules telles que définies dans la revendication 7 ou 8, dans des conditions appropriées pour l'expression du polypeptide et éventuellement récupérer ensuite le polypeptide.

10. Procédé selon la revendication 9, dans lequel les cellules sont ouvertes et le polypeptide est récupéré en utilisant la chromatographie d'affinité.

11. Composition pharmaceutique comprenant un allergène tel que défini dans l'une quelconque des revendications 1 à 4 et/ou un polynucléotide tel que défini dans la revendication 5.

12. Utilisation d'un allergène tel que défini dans l'une quelconque des revendications 1 à 4, ou d'un polynucléotide tel que défini dans la revendication 5, pour la préparation d'un médicament destiné au traitement ou à la prévention d'une allergie au pollen des mauvaises herbes.

13. Utilisation selon la revendication 12, dans laquelle le médicament est administré à un individu à désensibiliser.

14. Kit utile pour le diagnostic, le traitement et/ou la prévention d'un trouble allergique, comprenant un allergène tel que défini dans l'une quelconque des revendications 1 à 4 et/ou un polynucléotide tel que défini dans la revendication 5.

15. Utilisation d'un allergène tel que défini dans l'une quelconque des revendications 1 à 4 ou d'un polynucléotide tel que défini dans la revendication 5 pour le diagnostic d'une allergie au pollen des mauvaises herbes.
